# EUROPEAN PATENT SPECIFICATION

(11) **EP 2 219 643 B1**
(45) Date of publication and mention of the grant of the patent: **16.05.2012**
(21) Application number: 08850197.8
(22) Date of filing: 12.11.2008
(51) Int. Cl.: A61K 31/366, A61K 31/495, A61K 31/498, A61K 31/724, A61K 9/00

(54) **ANTHELMINTIC PASTE COMPRISING PRAZIQUANTEL, A MACROLIDE LACTONE, CYCLODEXTRIN AND A THICKENER**
ANTHELMINTISCHE PASTE MIT PRAZIQUANTEL, EINEM MAKROLID-LACTON, CYCLODEXTRIN UND EINEM VERDICKUNGSMITTEL
PÂTE ANTHELMINTIQUE COMPRENANT DU PRAZIQUANTEL, UNE LACTONE DE MACROLIDE, UNE CYCLODEXTRINE ET UN ÉPAISSISSANT

(30) Priority: 15.11.2007 EP 07120767
(43) Date of publication of application: 25.08.2010
(73) Proprietor: Novartis AG, 4056 Basel (CH)
(72) Inventor: WIELAND-BERGHAUSEN, Susanne, Christine, 79541 Lörrach (DE); OPPEL, Katrin, CH-4053 Basel (CH); HAAS, Stefan, CH-4322 Mumpf (CH)
(74) Representative: Liphardt, Bernd
(86) International application number: PCT/EP2008/065335
(87) International publication number: WO 2009/062939

(56) References cited:
- EP-A- 0 930 077
- WO-A-2004/000034
- WO-A-2005/062782
- WO-A-2006/011051
- AU-A4- 2007 100 254

## Description

The present invention relates to novel oral compositions for the control and treatment of endoparasitic infestations in warm-blooded animals, in particular in companion animals such as cats and dogs.

Praziquantel, macrocyclic lactones or combinations thereof are frequently used in the control and treatment of helminthes in warm-blooded animals. The active ingredients may be applied, for example, by drench or injection or preferably orally, for example in powder or tablet form. However, the handling of powders or granules is in general cumbersome and tablets often cause problems because animals such as dogs or especially cats don't tend to take them up voluntarily.

US patent No. 5,824,653 discloses a parasiticidal paste for oral application to horses comprising praziquantel and abamectin. The paste is an oil/water emulsion with the abamectin being dissolved during the manufacturing process. While this paste may be acceptable in the treatment of horses, it is unsuitable for the application to smaller animals, for example, because of an insufficient dosing ability, paste stability and palatability. A similar paste formulation of ivermectin and praziquantel is disclosed in AU 2007/100254.

WO 2004/000034 discloses non-aqueous paste formulations of a macrocyclic lactone and praziquantel. WO 2005/062782 further summarizes the state of the art of paste formulations comprising a macrocyclic lactone and praziquantel. EP 0930077 and WO 2006/011051 disclose taste masked solid compositions comprising a pharmaceutically active ingredient that is complexed with a cyclodextrin.

For example, a suitable palatability is crucial for an oral drug application to a cat or dog. While, in humans, medicaments may be administered relatively easily because the discipline and the desire to recover in human patients can be relied upon, dogs and especially cats refuse to take them orally, as soon as a pharmaceutical active ingredient has a taste which is unpleasant to the animal, whether because it is bitter or has some other unpleasant taste or is simply alien to the animal. Praziquantel is very problematic in this respect because of its bad smell and bitter taste.

Moreover, a veterinary composition for dogs and cats should be useable also by the pet owner at home. This requires on the one hand a sufficient paste stability and, in addition, the ability of the composition to be dosed accurately in small portions. The known oil in water emulsions always face the issue of phase separation and are thus inconvenient for home use because of their low stability. In addition, praziquantel and also macrocyclic lactones such as abamectin have a comparably low dosage safety margin which underlines the importance of an accurate dosing.

US patent No. 7,144,878 therefore proposes to replace praziquantel by pyrantel or morantel in a similar oral composition, although praziquantel clearly is the preferred active ingredient with respect to the antheimintic activity spectrum.

The problem to be solved within the present invention therefore was to find new improved anthelmintically active paste formulations for use in companion animals like cats and dogs which overcome the above-mentioned drawbacks. The problem is solved by providing a water-based suspension of the active ingredients in form of a paste comprising a cyclodextrin. Said novel pastes in general have a low content of or are even devoid of organic solvents.

The present invention therefore in one embodiment relates to an anthelmintic paste in form of an aqueous suspension, comprising
a veterinarily effective amount of praziquantel;
a veterinarily effective amount of a macrocyclic lactone selected from avermectins, milbemycins and derivatives thereof;
a cyclodextrin; a thickener; and
water; wherein the water contents of the anthelmintic paste is ≥50% by weight, based on the weight of the entire formulation; and wherein the anthelmintic paste is devoid of a surfactant.

The first active ingredient of the aqueous suspensions of the invention is praziquantel, 2-(cyclohexylcarbonyl)-1,2,3,6,7,11b-hexahydro-4H-pyrazino[2,1-a]isoquinoline-4-one,which has the following structure:

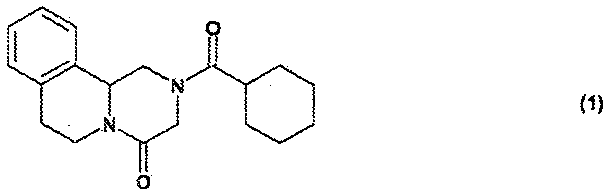

The compound may be employed in form of the racemate or in form of one of its isomers, the (s)-isomer or the levo-(r)-isomer.

It is beneficial to employ an anthelmintic formulation of the present invention wherein praziquantel is present in an amount of from 0.5 to 20% by weight, preferably, from 0.5 to 15% by weight, more preferably from 1.0 to 10.0% by weight, and in particular from 2.0 to 7.5% by weight, based on the entire formulation.

The aqueous suspensions of the present invention comprise a veterinarily effective amount of a second active ingredient which is a macrocyclic lactone selected from avermectins, milbemycins and derivatives thereof.

Non-limiting examples of macrocyclic lactone compounds are Ivermectin, Doramectin, Moxidectin, Selamectin, Emamectin, Eprinomectin, Milbemectin, Abamectin, Milbemycin oxime, Nemadectin, and derivatives thereof, in free form or in the form of a physiologically acceptable salt.

In the context of the invention, a preferred group of macrocyclic lactones is represented by compounds of formula 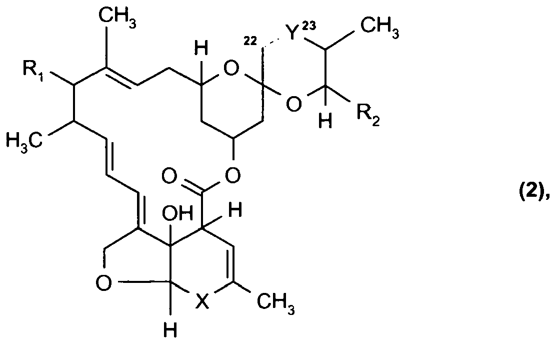 wherein X is -C(H)(OH)-; -C(O)-; or -C(=N-OH)-; Y is -C(H₂)-; =C(H)- ; -C(H)(OH)-; or -C(=N-OCH₃)-; R₁ is hydrogen or one of radicals 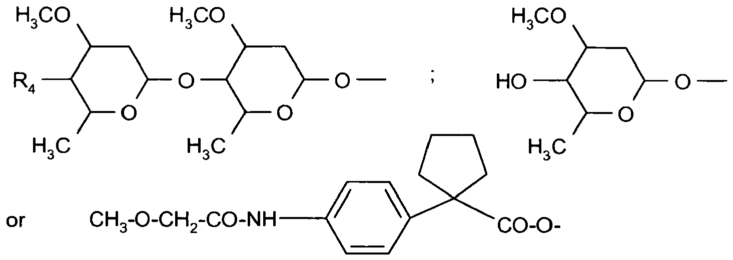 R₄ is hydroxyl, -NH-CH₃ or -NH-OCH₃; and R₂ is hydrogen, -CH₃, -C₂H₅, -CH(CH₃)₂, -CH(CH₃)-C₂H₅, -C(CH₃)=CH-CH(CH₃)₂ or cyclohexyl; and if the bond between atoms 22 and 23 represents a double bond the carbon atom in 23-position is unsubstituted so that Y is =C(H)-, or if is the bond between atoms 22 and 23 is a single bond the carbon atom in 23-position is unsubstituted or substituted by hydroxy or by the group =N-O-CH₃ so that Y is -C(H₂)-; -C(H)(OH)-; or -C(=N-OCH₃)-; in free form or in the form of a physiologically acceptable salt.

Typical and especially preferred representatives of compounds of formula (2) are:
1) Ivermectin is 22,23-Dihydroabamectin; 22,23-dihydroavermectin B 1; or 22,23-dihydro C-076B 1, wherein X is -C(H)(OH) ; Y is -C(H₂)-; R₁ is the radical 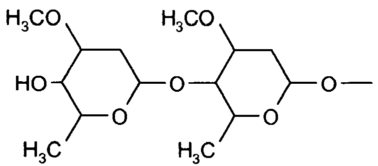 R₂ is either -CH(CH₃)-CH₃ or -CH(CH₃)-C₂H₅ and the bond between atoms 22 and 23 represents a single bond. Ivermectin is known from US-4,199,569.
2) Doramectin is 25-Cyclohexyl-5- O- demethyl-25-de(1-methylpropyl)avermectin A 1a, wherein X is -C(H)(OH)-; Y is =C(H)-; R₁ is the radical 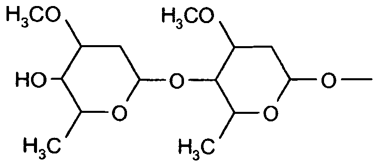 R₂ is cyclohexyl and the bond between atoms 22 and 23 represents a double bond. Doramectin is known from US-5,089,480.
3) Moxidectin, is [6R ,23E, 25S(E)]-5-O-Demethyl-28-deoxy-25-(1,3-dimethyl-1-butenyl)-6,28-epoxy-23-(methoxy im ino)milbemycin B, wherein X is -C(H)(OH)- ; Y is - C(=N-OCH₃₎-; R₁ is hydrogen; R₂ is -C(CH₃)=CH-CH(CH₃)₂; and the bond between atoms 22 and 23 represents a single bond. Moxidectin, is known from EP-0,237,339 and US-4,916,154.
4) Selamectin is 25-cyclohexyl-25-de(1-methylpropyl)-5-deoxy-22,23-dihydro-5-(hydroxyimino)avermectin B1 monosaccharide and thus a compound of formula (2), wherein X is -C(=N-OH)-; Y is -C(H₂)-; R₁ is the radical 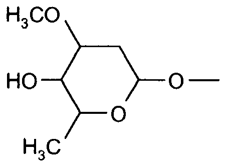 R₂ is cyclohexyl; and the bond between atoms 22 and 23 represents a single bond. Selamectin is known e.g. from: ECTOPARASITE ACTIVITY OF SELAMECTIN; A novel endectocide for dogs and cats. A Pfizer Symposium, held in conjunction with The 17th international Conference of the World Association for the Advancement of Veterinary Parasitology, 19 August 1999. Copenhagen, Denmark.
5) Emamectin is (4primeprime R)-5- O -demethyl-4primeprimedeoxy-4primeprime-(methlamino) avermectin A 1a and (4primeprime R)-5- O -demethyl-25-de(1-methylpropyl)-4primeprime-deoxy-4primeprime-(methylamino)-25-(1-methylethyl)avermectin A 1 a (9:1), wherein X is -C(H)(OH) ; Y is =C(H)-; R₁ is 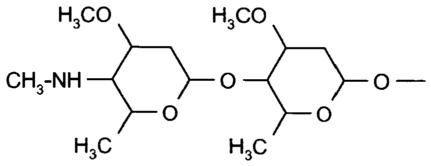 R₂ is -CH(CH₃)-CH₃, or -CH(CH₃)-C₂H₅, and the bond between atoms 22 and 23 represents a double bond. Emamectin is known from US-4,874,749.
6) Eprinomectin is (4primeprime R)-4primeprime- epi- (acetylamino)-4primeprime-deoxyavermectin B 1, wherein X is -C(H)(OH)-; Y is =C(H)- ; R₁ is the radical 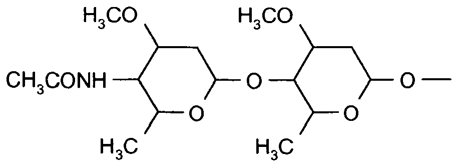 R₂ is -CH(CH₃)-CH₃, or -CH(CH₃)-C₂H₅, and the bond between atoms 22 and 23 represents a double bond. Eprinomectin is known from US-4,427,663.
7) Milbemectin is (6R,25R)-5-O-demethyl-28-deoxy-6,28-epoxy-25-methylmilbemycin, wherein X is -C(H)(OH)-; Y is -C(H₂)-; R₁ is hydrogen; R₂ is -CH₃, or -C₂H₅; and the bond between atoms 22 and 23 represents a single bond. Milbemectin is known from US-3,950,360.
8) Abamectin is Avermectin B 1 which is also named 5- O- demethylavermectin A 1 a and 5- O- demethyl-25-de(1-methylpropyl)-25-(1-methylethyl)avermectin A 1a (4:1), wherein X is -C(H)(OH)-; Y is =C(H)-; R₁ is the radical 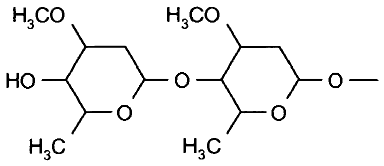 R₂ is -CH(CH₃)-CH₃, or -CH(CH₃)-C₂H₅; and the bond between atoms 22 and 23 represents a double bond. Abamectin is known from US-4,310,519.
9) Milbemycin oxime is a compound of formula (2), wherein X is -C(=N-OH)-; Y is -C(H₂)-; R₁ is hydrogen; R₂ is -C₂H₅ or -CH₃, and the bond between atoms 22 and 23 represents a single bond. Milbemycin oxime is known from US-4,547,520.
10) The compound of the formula (2) wherein X is -C(H)(OH)-; Y is -C(H₂)-; R₁ is the radical 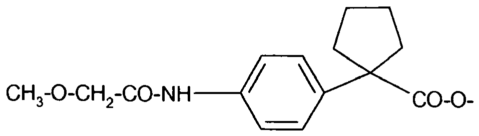 R₂ is -CH₃ or C₂H₅, and the bond between atoms 22 and 23 represents a single bond. This compound is known from WO 01/83500.
11) Nemadectin is antibiotic S-541 A; also named [6R, 23S, 25S,(E)]-5- O- Demethyl-28-deoxy-25-(1,3-dimethyl-1-butenyl)-6,28-epoxy-23-hydroxymilbemycin B; wherein X is =CH-OH; Y is -C(H₂)-; R₁ is hydrogen; R₂ is -C(CH₃)=CH-CH(CH₃)₂, and the bond between atoms 22 and 23 represents a single bond. Nemadectin is known from US-4,869,901.

The avermectins, milbemycins and milbemycin oximes of the formula (2) above are often employed as a mixture of two compounds, which are identical with the exception of the radical R₂. For example, Ivermectin is a mixture of two compounds as described above, the major one having a radical R₂ which is sec.-butyl, the other one having a radical R₂ which is isopropyl. The ratio between the sec.-butyl compound and the isopropyl compound is, for example, about 80:20. Likewise, Milbemycine oxime is a mixture of two above described compounds, the major one having a radical R₂ which is ethyl, the other one having a radical R₂ which is methyl. The ratio between the ethyl compound and the methyl compound is, for example, about 80:20. Most preferred macrocyclic lactones within the formulations of the present invention are ivermectin or, in particular, milbemycin oxime.

The macrocyclic lactone is present in the anthelmintic formulations of the invention in an amount of, for example, from 0.1 to 10% by weight, preferably, from 0.1 to 8% by weight, more preferably from 0.5 to 5.0% by weight, and in particular from 1.0 to 2.5% by weight, based on the entire formulation.

Cyclodextrins for use according to the present invention include, for example, unsubstituted or substituted cyclodextrins having from 6 to 12 glucose units, for example α-, β- and γ-cyclodextrins and derivatives thereof. Suitable cyclodextrin derivatives are, for example, C₁-C₆-alkyl and hydroxyl-C₁-C₆-alkyl derivatives, for example hydroxpropyl- β-cyclodextrin or hydroxethyl-β-cyclodextrin. In a preferred embodiment the cyclodextrin is a β-cyclodextrin or a derivative thereof, in particular naturally occurring β-cylclodextrin. The cyclodextrins may be amorphous or crystalline.

The concentration of the cyclodextrin in the formulations of the invention may vary within wide limits dependant, for example, on the concentration of the active ingredients. In general, the cyclodextrin is present in the inventive formulations in an amount of, for example, from 0.5 to 40% by weight, preferably from 1.0 to 20% by weight, more preferably from 1.5 to 10% by weight, and in particular from 2.0 to 7.5% by weight, based on the entire formulation.

Suitable thickeners useful in the formulations of the present invention include in principle all thickeners being acceptable for oral application in animals. Examples of suitable thickeners are carrageen, xanthan gum, polyvinylpyrrolidones, carbomers (B.F. Goodrich), cornstarch, oat meal flour, microcrystalline cellulose (Avicel), hydroxyethyl cellulose, edible clay, silicon dioxide or combinations thereof. Preferred thickeners are xanthan gum, microcrystalline cellulose, polyvinylpyrrolidone, or a mixture of two or three thereof. Particular preferred thickeners are xanthan gum or a mixture of xanthan gum and a polyvinylpyrrolidone or a mixture of xanthan gum, a polyvinylpyrrolidone and a microcrystalline cellulose.

The thickener is present in the formulations of the present invention, for example, in an amount of from 0.1 to 10% by weight, preferably from 0.5 to 7.5% by weight, and in particular from 1 to 5% by weight, based on the entire formulation.

The anthelmintic aqueous suspensions of the present invention, in addition, may contain further veterinary acceptable ingredients, for example, humectants, preservatives, pH-adjusting agents, colors, flavors, binders, fillers or dispersing agents. Said veterinarily acceptable ingredients are known to those skilled in the art of veterinary formulation technology. A preferred humectant is glycerol. Suitable flavors within the composition of the invention are, for example, artificial beef flavor or food extracts such as desiccated liver, malt extract or fish meal. Useful preservatives are, for example benzyl alcohol, benzoic acid or benzoates such as sodium benzoate or mixtures thereof. Suitable pH-adjusting agents are, for example bases or, in particular, acids. An example of an orally acceptable color is titanium dioxide or iron oxide red.

The anthelmintic formulations of the present invention are water-based suspensions which are devoid of any organic solvent dissolving the praziquantel and/or the macrocyclic lactone. In particular, the formulations of the present invention are devoid of (i) lubricants such as oils, for example vegetable oils, mineral oils, or Miglyol^{®} brands, for example propylene glycol dicaprylate/dicaprate (Miglyol® 840); (ii) solvents such as polyglycols, for example a polyethylene or polypropylene glycol, glycol ethers, for example ethylene glycol monomethyl- or monoethyl ether or diethylene glycol monomethyl- or monoethylether; or glycerol formal; and (iii) surfactants such as lauryl sulfate or polysorbate 80. The water content of the paste formulations of the present invention is ≥ 50% by weight and in particular ≥ 60% by weight, in each case based on the weight of the entire formulation.

Preferred embodiments of the anthelmintic suspension formulation according to the present invention are as follows:
(1) An aqueous suspension formulation in form of a paste comprising

| | |
|---|---|
| 0.5 to 20% by weight | of praziquantel; |
| 0.1 to 8% by weight | of a macrocyclic lactone, wherein the above-given meanings and preferences apply; |
| 0.5 to 40% by weight | of a cyclodextrin; |
| 0.1 to 10% by weight | of a thickener; and |
| ad 100% by weight | water; |

(2) an aqueous suspension formulation in form of a paste comprising

| | |
|---|---|
| 1.0 to 10% by weight | of praziquantel; |
| 0.5 to 5% by weight | of a macrocyclic lactone, wherein the above-given meanings and preferences apply; |
| 1.0 to 20% by weight | of a cyclodextrin; |
| 0.5 to 7.5% by weight | of a thickener; and |
| ad 100% by weight | water; |

(3) an aqueous suspension formulation in form of a paste comprising

| | |
|---|---|
| 2.0 to 7.5% | by weight of praziquantel; |
| 1.0 to 2.5% | by weight of a macrocyclic lactone, wherein the above-given meanings and |
| | preferences apply; |
| 2.0 to 7.5% by weight | of a cyclodextrin; |
| 1.0 to 5.0% by weight | of a thickener; and |
| ad 100% by weight | water; |

(4) each of the aqueous suspension formulations (1), (2) or (3) above, wherein the macrocyclic lactone is milbemycin oxime;
(5) each of the aqueous suspension formulations (1), (2), (3) or (4) above, wherein the cyclodextrin is β-cyclodextrin or a derivative thereof;
(6) each of the aqueous suspension formulations (1) to (5) above, wherein the water contents is ≥ 50% by weight, or preferably ≥ 60% by weight, based on the entire formulation;
(7) each of the aqueous suspension formulations (1) to (6) above, which comprises one or more additional components selected from the group consisting of humectants, preservatives, pH-adjusting agents, colors and flavors;
(8) (1) An aqueous suspension formulation in form of a paste consisting of

| | |
|---|---|
| 1.0 to 10% by weight | of praziquantel; |
| 0.5 to 5% by weight | of milbemycin oxime; |
| 1.0 to 20% by weight | of a cyclodextrin; |
| 0.5 to 7.5% by weight | of thickeners; |
| 1.0 to 10% by weight | of humectants; |
| 0.5 to 5% by weight | of preservatives; |
| 2.5 to 20% by weight | of flavors; |
| 0 to 5.0% by weight | colors; and |
| ad 100% by weight | water and acid. |

The suspension formulations of the present invention may be prepared by methods known per se in the art of suspension technology. As a general principle, water is provided, and the components are added and dispersed therein consecutively, preferably in a one pot process. The order of components to be added to the water is in principle not critical. However, it has turned out that it is advantageous to first add the cyclodextrin to the water and disperse and/or partly dissolve it in the water while stirring.

A suitable process for the manufacture of the suspension formulation of the present invention therefore comprises
(i) providing the water and optionally further ingredients which are soluble in water, such as pH-adjusting agents, preservatives or humectants,
(ii) adding the cyclodextrin and disperse and/or partly dissolve it in the water,
(iii) adding the praziquantel and disperse it in the water/cyclodextrin mixture,
(iv) dispersing the macrocyclic lactone, optional further ingredients and a thickener consecutively, and
(v) stirring the dispersion unless a homogeneous paste is formed.

In case that further ingredients being present in the water in step (i), it may be advisable to heat up the water to a temperature of, for example, from above room temperature to about 80°C and/or to decrease the pH value of the water to a value of, for example from 1.5 to 6, preferably from 2 to 5, by the addition on an acid, for example, in order to accelerate solubilization of said ingredients in the water. The cyclodextrin and afterwards the praziquantel are added and the stirring is continued for some time after each addition, for example for from 15 minutes to 1 hour, before the rest of the paste ingredients are added. The weight ratio of praziquantel to cyclodextrin is advantageously chosen to be from about 1:1 to 1:6, preferably from about 1:1 to 1:5 and in particular from about 1:1 to 1:2. As mentioned before, the order of addition of the further components is not critical. For example, the macrocyclic lactone is added, followed by optionally additional components like color, flavor and the like and thickeners to finally adjust the viscosity and specific gravity of the pastes to a desired value.

A suitable specific gravity of the pastes of the present invention is, for example, from about 1.05 to 1.15, preferably from about 1.07 to 1.12, and in particular from 1.08 to 1.10. The pH-value of the final suspension paste is preferably between 3 and 7, preferably 4 to 5.5, and may be adjusted by the addition of suitable bases or acids. Advantageously, the viscosity of the paste formulation is adjusted so that sedimentation is prevented on the one hand and easy applicability and exact dosing are maintained on the other hand.

The paste compositions of the present invention are preferably thixotropic, which means that the viscosity thereof decreases with an increase of the shear stress applied. The thixotropic properties of the pastes of the present invention increase the ease of dosing, especially when applying the paste by means of a pipette.

The aqueous suspension pastes of the present invention are suitable for controlling pathogenic endoparasites in warm-blooded animals such as, for example, companion animals, in particular dogs or especially cats; they have a favorable toxicity to warm-blooded species. They are effective against all or individual development stages of the pests. Pathogenic endoparasites include cestodes, trematodes, nematodes and acanthocephala.

The aqueous suspension formulations of the present invention are applied to the animals preferably by means of a pipette. Pipettes offer the advantage e.g. of exact dosing. Filling the exact dose of paste needed into a pipette and applying the paste directly into the mouth, assures that the animals are getting the exact dose. A prerequisite for the use of the suspension pastes of the present invention in pipettes especially for home-use is their excellent stability and shelf-life . No noticeable phase separation, inhomogeneity or change in physical parameters such as specific gravity is detectable even after long periods of time following their preparation.

Moreover, the aqueous suspension pastes of the present invention have an improved palatability meaning the voluntary acceptance or ingestion of the pharmaceutical composition by warm-blooded animals, in particular by dogs or especially cats.

A suitable acceptance test on cats may be performed as follows. Throughout the study conduct, 1 ml portions of all tested oral paste formulations are administered as follows. Initially, a small amount of paste is applied on the lips / nose of the animal and the acceptance is rated as follows: High acceptance (3 points) is reached if the cat spontaneously licks and swallows the paste and the complete dose is administered without inserting the pipette into the mouth. Satisfactory acceptance (2 points) is achieved when the paste is not spontaneously licked, but the pipette must be inserted in the cat's mouth and the dose is administered entirely. The cat might shake its head and chew during and after administration, but neither hypersalivation nor vomiting over the post-dose 5 minutes is accepted. For a poor acceptance (1 point) the criteria are the same as for satisfactory acceptance, but hypersalivation and/or excitation might be observed and vomiting can occur within 5 minutes after paste application. The worst rating, failed acceptance (0 points) is given if the dose is not administered at all due to incidents such as head withdrawal or scratch attempt or if the paste is entirely rejected after insertion into the mouth. This might also involve hypersalivation and vomiting.

The following examples illustrate the present invention.

**Example 1**: An orally applied, homogeneous aqueous suspension paste, which has the following ingredients:

| | Amount (%w/v) |
|---|---|
| Praziquantel | 2.5 % |
| Milbemycin oxime | 1.0 % |
| β-Cyclodextrin | 2.5 % |
| Polyvinylpyrrolidone K30 | 1.0 % |
| Sodium benzoate | 0.4 % |
| Benzyl alcohol | 1.0 % |
| Artificial beef flavor | 10.0 % |
| Titanium dioxide | 2.0 % |
| Glycerol water free | 5.0 % |
| Xanthan gum | 1.0 % |
| 0.01M HCl | ad 100.0 % |

is prepared by the following process:
(i) sodium benzoate, benzyl alcohol and glycerol are dissolved in the 0.01 M hydrochloric acid solution;
(ii) following the addition and dissolution/dispersion of the β-cyclodextrin and polyvinylpyrrolidon K30, the praziquantel is added and homogeneously dispersed;
(iii) milbemycin oxime, artificial beef flavour and titanium dioxide are added and dispersed before finally xanthan gum is added and dispersed to yield a homogeneous, aqueous suspension paste. The specific gravity of the final paste is 1.0814 g/ml.

**Example 2**: An orally applied, homogeneous aqueous suspension paste, which has the following ingredients:

| | Amount (%w/v) |
|---|---|
| Praziquantel | 5.0 % |
| Milbemycin oxime | 2.0 % |
| β-Cyclodextrin | 5.0 % |
| Polyvinylpyrrolidone K30 | 1.0 % |
| Sodium benzoate | 0.5 % |
| Benzyl alcohol | 1.25 % |
| Artificial beef flavor | 10.0 % |
| Glycerol water free | 5.0 % |
| Xanthan gum | 1.0 % |
| 0.01M HCl | ad 100.0 % |

is prepared using the process as described in Example 1 (specific gravity 1.0844 g/ml).

### Example 3: Paste stability test

The paste composition of Example 2 was stored in glass containers at different temperatures for up to 12 months and assay of both active ingredients followed by HPLC analysis at given time points (3-, 6- and 12-months). The Table 1 below shows the respective contents of praziquantel and milbemycin oxim in the composition in percent of the start value (= assay right after manufacture)

**Table 1: Content of praziquantel and milbemycin Oxime in % of start value**

| (A) Praziquantel: | | | | |
|---|---|---|---|---|
| Time | 5 °C | 25 °C | 30 °C | 40°C |
| 3 month | 102.20% | 102.52% | 100.63% | 101.36% |
| 6 month | 103.98% | 101.99% | 102.31% | 102.10% |
| 12 month | 103.56% | 102.73% | 102.62% | 100.94% |

| (B) Milbemycin oxim: | | | | |
|---|---|---|---|---|
| Time | 5 °C | 25 °C | 30 °C | 40°C |
| 3 month | 101.92% | 101.71% | 99.57% | 99.47% |
| 6 month | 102.77% | 100.32% | 100.43% | 98.83% |
| 12 month | 101.71% | 100.53% | 99.68% | 95.20% |

The results given in the Table above show that both active ingredients are chemically stable in the given formulation under the given conditions.

In addition to the chemical stability of both active ingredients, the paste did not show any macroscopic sedimentation or segregation during storage and the pH value of the paste formulation was stable at all investigated temperatures and time points as well.

### Example 4: Paste acceptance Test in cats

About 90 healthy cats, tested negative for heartworm, greater than or equal to six (6) months of age, ≥ 1 and ≤ 22 pounds in weight, of various sexes and breeds were included in the study.

Test material consisted of the oral paste formulation of Example 1 for cats less than 7 lbs of body weight and the oral paste of Example 2 for cats 7lbs to 22lbs. Both formulations provided a dose of 2mg/kg milbemycin oxime and 5 mg/kg praziquantel. Each cat was orally administered the paste by it's Owner/Agent at home on Study Days 0 and 21 via prefilled syringes prepared in the veterinary clinic on Study Day 0. The syringes were packaged in child resistant envelops for home storage. To assess cat Owner's response to administering the paste as compared to a tablet to cats, ¼ of a Pet Tab® (vitamin-mineral supplement) was administered by way of a pilling device on Study Day 27 ±2d as a control. The acceptability by the cat and administration by the Owner was assessed by analysis of responses provided by the Owner/Agent on the Owner/Agent Diary. This Diary was completed following administration of each test material.

The acceptability endpoint was the only variable evaluated and was assessed based on the cat's behavior. The Owner recorded responses on the Pet Owner Diary after each administration of paste or control product, and filled out the Owner Follow-up Questionaire after the last dosing.

The frequency and percent for each acceptance score for the paste versus the tablet is given in Tables 2-4.

**Table 2. Frequency and Percent for Each Acceptance Score for the Paste on day 0**

| **Score** | **Frequency** | **%** |
|---|---|---|
| 2=swallowed all | 51 | 64.88 |
| 1=swallowed part | 25 | 29.17 |
| 0=spat it all out | 8 | 5.95 |

**Table 3. Frequency and Percent for Each Acceptance Score for the Paste on day 21**

| **Score** | **Frequency** | **%** |
|---|---|---|
| 2=swallowed all | 58 | 69.05 |
| 1=swallowed part | 24 | 28.57 |
| 0=spat it all out | 2 | 2.38 |

**Table 4. Frequency and Percent for Each Acceptance Score for the Tablet on day 27**

| **Score** | **Frequency** | **%** |
|---|---|---|
| 2=swallowed all | 43 | 50.59 |
| 1 =swallowed part | 10 | 11.76 |
| 0=spat it all out | 32 | 37.65 |

Over 64% of the cats accepted all of the paste, whereas only 50% of the cats accepted all of the commonly used tablet.

Owner Response to Dosing; Paste compared to Tablet:
The percent for each ease of dose score for the paste and the tablet is given in Table 5 . Over 71% of the owners found it quite easy or very easy to administer the paste, whereas only 47% of the owners found it quite easy or very easy to administer the tablet.

**Table 5. Owner response to dosing**

| **Score** | **Paste** | | **Tablet** | |
|---|---|---|---|---|
| | **Frequency** | **%** | **Frequency** | **%** |
| 3= very easy | 48 | 28.07 | 26 | 29.89 |
| 2= quite easy | 74 | 43.27 | 15 | 17.24 |
| 1= difficult | 41 | 23.98 | 17 | 19.54 |
| 0= impossible | 8 | 4.68 | 29 | 33.33 |

## Claims

1. An anthelmintic paste in form of an aqueous suspension, comprising
a veterinarily effective amount of praziquantel;
a macrocyclic lactone selected from avermectins, milbemycins and derivatives thereof;
a cyclodextrin;
a thickener; and
water; wherein the water contents of the anthelmintic paste is ≥50% by weight, based on the weight of the entire formulation; and wherein the anthelmintic paste is devoid of a surfactant.

2. A paste according to claim 1, wherein the macrocyclic lactone is of formula 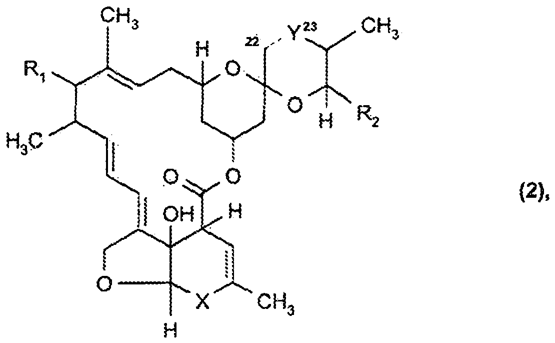 wherein X is -C(H)(OH)-, -C(O)-; or -C(=N-OH)-; Y is -C(H₂)-; =C(H)- ; -C(H)(OH)-; or -C(=N-OCH₃)-: R₁ is hydrogen or one of radicals 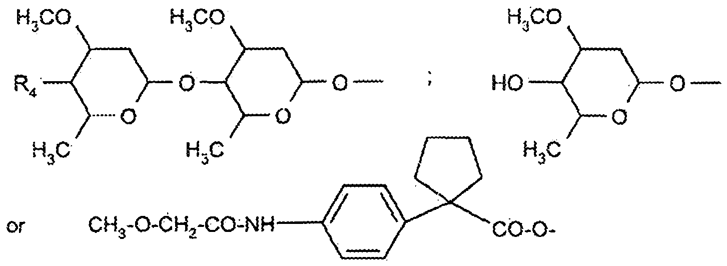 R₄ is hydroxyl, -NH-CH₃ or -NH-OCH₃; and R₂ is hydrogen, -CH₃, -C₂H₅, -CH(CH₃)₂, -CH(CH₃)-C₂H₅, -C(CH₃)=CH-CH(CH₃)₂ or cyclohexyl; and if the bond between atoms 22 and 23 represents a double bond the carbon atom in 23-position is unsubstituted so that Y is =C(H)-, or if is the bond between atoms 22 and 23 is a single bond the carbon atom in 23-position is unsubstituted or substituted by hydroxy or by the group =N-O-CH₃ so that Y is -C(H₂)-; -C(H)(OH)-; or -C(=N-OCH₃)-; in free form or in the form of a physiologically acceptable salt.

3. A paste according to claim 1 or 2, wherein the macrocyclic lactone is ivermectin or milbemycin oxime, in particular milbemycin oxime.

4. A paste according to any one of claims 1 to 3, comprising
| | |
|---|---|
| 0.5 to 20% by weight | of praziquantel; |
| 0.1 to 8% by weight | of a macrocyclic lactone; |
| 0.5 to 40% by weight | of a cyclodextrin; |
| 0.1 to 10.0% by weight | of a thickener; and |
| ad 100% by weight | water. |

5. A paste according to any one of claims 1 to 4, comprising
| | |
|---|---|
| 2.0 to 7.5% by weight | of praziquantel; |
| 1.0 to 2.5% by weight | of a macrocyclic lactone, |
| 2.0 to 7.5% by weight | of a cyclodextrin; |
| 1.0 to 5.0% by weight | of a thickener; and |
| ad 100% by weight | water. |

6. A paste according to claim 4 or 5, wherein the macrocyclic lactone, is milbemycin oxime.

7. A paste according to any one of claims 1 to 6, comprising one or more additional components selected from the group consisting of humectants, preservatives, ph-adjusting agents, colors and flavors.

8. A paste according to any one of claims 1 to 7, comprising a water contents of ≥60% by weight, based on the entire formulations.

9. A paste according to any one of claims 1 to 8, wherein the thickener is xanthan gum or is a mixture of xanthan gum and polyvinylpyrrolidone or is a mixture of xanthan gum, polyvinylpyrrolidone and microcrystalline cellulose.

10. A paste according to claim 2, consisting of
| | |
|---|---|
| 1.0 to 10% by weight | of praziquantel; |
| 0.5 to 5% by weight | of milbemycin oxime; |
| 1.0 to 20% by weight | of a cyclodextrin; |
| 0.5 to 7.5% by weight | of thickeners; |
| 1.0 to 10% by weight | of humectants; |
| 0.5 to 5% by weight | of preservatives; |
| 2.5 to 20% by weight | of flavors; |
| 0 to 5.0% by weight | colors; and |
| ad 100% by weight | water and acid. |

11. A paste according to any one of claims 1 to 11, **characterised in that** it has a specific gravity from 1.05 to 1.15.

12. A paste according to any one of claim 1 to 11, **characterised in that** it is thixotropic.

13. Process for the manufacture of an anthelmintic paste according to any one of claims 1 to 12, comprising
(i) providing the water and optionally further ingredients which are water-soluble,
(ii) adding the cyclodextrin and disperse and/or partly dissolve it in the water,
(iii) adding the praziquantel and disperse it in the water/cyclodextrin mixture,
(iv) dispersing the macrocyclic lactone, optional further ingredients and a thickener consecutively, and
(v) stirring the dispersion unless a homogeneous paste is formed.

14. Use of an anthelmintic paste according to any one of claim 1 to 12 in the manufacture of a medicament for the treatment of endoparasites in warm-blooded animals, in particular in dogs and cats.

15. Use of an anthelmintic paste according to any one of claim 1 to 12 in the preparation of a medicament for controlling endoparasites in warm-blooded animals, in particular in dogs and cats.

## Patentansprüche

1. Anthelmintische Paste in Form einer wässrigen Suspension, umfassend eine veterinärisch effektive Menge an Praziquantel;
ein makrozyklisches Lakton ausgewählt aus Avermectinen, Milbemycinen und Derivaten davon;
ein Zyklodextrin;
ein Verdickunsgmittel; und
Wasser; wobei der Wassergehalt der anthelmintischen Paste ≥50 Gewichts-%, basierend auf dem Gewicht der gesamten Formulierung, ist; und wobei die anthelmintische Paste frei von Tensid ist.

2. Paste nach Anspruch 1, wobei das makrozyklische Lakton von der Formel 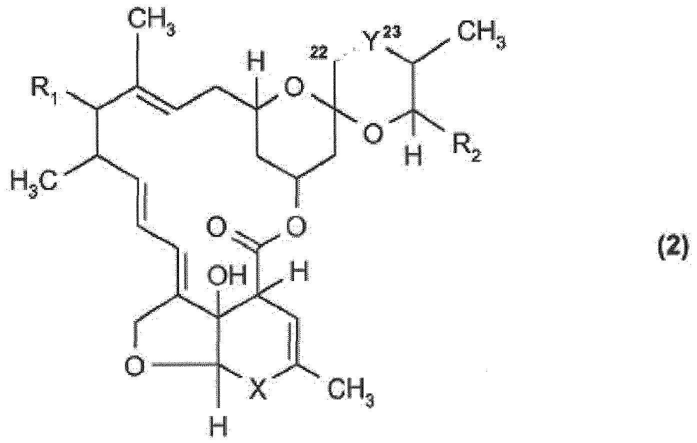 ist,
wobei X -C(H)(OH)-; -C(O)-; oder -C(=N-OH)- ist; Y -C(H₂)-; =C(H)-; - C(H)(OH)-; oder -C(=N-OCH₃)- ist; R₁ Wasserstoff oder eines der Radikale 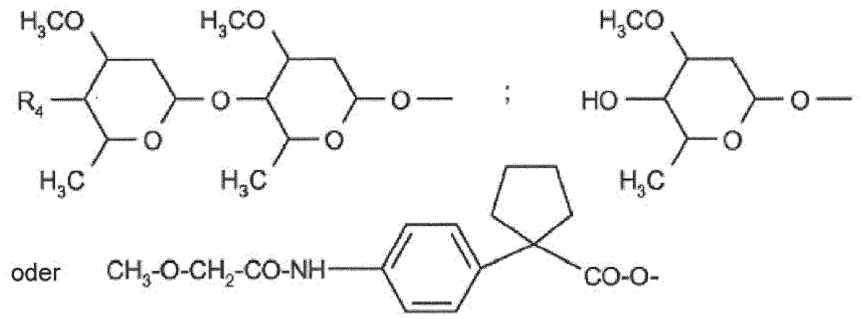 ist,
R₄ Hydroxyl, -NH-CH₃ oder -NH-OCH₃ ist; und R₂ Wasserstoff, -CH₃, -C₂H₅, CH(CH₃)₂, -CH(CH₃)-C₂H₅, -C(CH₃)=CH-CH(CH₃)₂ oder Zyklohexyl ist; und falls die Bindung zwischen den Atomen 22 und 23 eine Doppelbindung darstellt, das Kohlenstoffatom an der 23er-Position unsubstituiert ist, so dass Y =C(H)- ist oder falls die Bindung zwischen den Atomen 22 und 23 eine Einfachbindung darstellt, das Kohlenstoffatom an der 23er-Position unsubstituiert oder durch ein Hydroxy oder durch die Gruppe =N-O-CH₃ substituiert ist, so dass Y -C(H₂)-; -C(H)(OH)-; oder -C(=N-OCH₃)- ist; in der freien Form oder in der Form eines physiologisch akzeptablen Salzes.

3. Paste nach Anspruch 1 oder 2, wobei das makrozyklische Lakton Ivermectin oder Milbemycinoxim, insbesondere Milbemycinoxim, ist.

4. Paste nach einem der Ansprüche 1 bis 3, umfassend
0,5 bis 20 Gewichts-% von Praziquantel;
0,1 bis 8 Gewichts-% von einem makrozyklischem Lakton;
0,5 bis 40 Gewichts-% von einem Zyklodextrin;
0,1 bis 10,0 Gewichts-% von einem Verdickungsmittel; und
aufgefüllt auf 100 Gewichts-% Wasser.

5. Paste nach einem der Ansprüche 1 bis 4, umfassend
2,0 bis 7,5 Gewichts-% von Praziquantel;
1,0 bis 2,5 Gewichts-% von einem makrozyklischem Lakton;
2,0 bis 7,5 Gewichts-% von einem Zyklodextrin;
1,0 bis 5,0 Gewichts-% von einem Verdickungsmittel; und
aufgefüllt auf 100 Gewichts-% Wasser.

6. Paste nach einem der Ansprüche 4 oder 5, wobei das makrozyklische Lakton Milbemycinoxim, ist.

7. Paste nach einem der Ansprüche 1 bis 6, umfassend eine oder mehrere zusätzliche Komponenten ausgewählt aus der Gruppe bestehend aus Feuchthaltemitteln, Konservierungsstoffen, pH-anpassenden Werkstoffen, Farbstoffen und Geschmackstoffen.

8. Paste nach einem der Ansprüche 1 bis 7, umfassend einen Wassergehalt von ≥60 Gewichts-%, basierend auf der gesamten Formulierung.

9. Paste nach einem der Ansprüche 1 bis 8, wobei das Verdickungsmittel Xanthangummi oder eine Mischung aus Xanthangummi und Polyvinylpyrrolidon oder eine Mischung aus Xanthangummi, Polyvinylpyrrolidon und mikrokristalliner Zellulose ist.

10. Paste nach Anspruch 2, bestehend aus
1,0 bis 10 Gewichts-% von Praziquantel;
0,5 bis 5 Gewichts-% von Milbemycinoxim;
1,0 bis 20 Gewichts-% von einem Zyklodextrin;
0,5 bis 7,5 Gewichts-% von Verdickungsmitteln;
1,0 bis 10 Gewichts-% von Feuchthaltemitteln;
0,5 bis 5 Gewichts-% von Konservierungsmitteln;
2,5 bis 20 Gewichts-% von Geschmackstoffen;
0 bis 5,0 Gewichts-% von Farbstoffen; und
aufgefüllt auf 100 Gewichts-% Wasser und Säure.

11. Paste nach einem de Ansprüche 1 bis 11, **dadurch gekennzeichnet, dass** sie eine spezifische Gravitation von 1,05 bis 1,15 hat.

12. Paste nach einem der Ansprüche 1 bis 11, **dadurch gekennzeichnet, dass** sie thixotrop ist.

13. Verfahren zur Herstellung einer anthelmintischen Paste nach einem der Ansprüche 1 bis 12, umfassend
(i) Bereitstellen des Wassers und gegebenenfalls weiteren Inhalststoffen, welche wasserlöslich sind,
(ii) Zugeben des Zyklodextrins und es in dem Wasser dispergieren und/oder teilweise auflösen,
(iii) zugeben des Praziquantels und es in der Wasser/Zyklodextrinmischung dispergieren,
(iv) Dispergieren des makrozyklischen Laktons, gegebenenfalls weiterer Inhaltstoffe und anschließend eines Verdickungsmittels und
(v) Rühren der Dispersion bis sich eine homogene Paste bildet.

14. Verwendung einer anthelmintischen Paste nach einem der Ansprüche 1 bis 12 in der Herstellung eines Medikaments zur Behandlung von Endoparasiten in warmblütigen Tieren, insbesondere in Hunden und Kratzen.

15. Verwendung einer anthelmintischen Paste nach einem der Ansprüche 1 bis 12 in der Zubereitung eines Medikaments zur Kontrolle von Endoparasiten in warmblütigen Tieren, insbesondere in Hunden und Katzen.

## Revendications

1. Pâte anthelminthique se présentant sous forme d'une suspension aqueuse comprenant :
une quantité efficace d'un point de vue vétérinaire de praziquantel;
une lactone macrocyclique choisie parmi les avermectines, les milbemycines et
leurs dérivés ;
une cyclodextrine ;
un épaississant; et
de l'eau ; **caractérisée en ce que** la teneur en eau de la pâte anthelminthique est ≥ 50% en poids, sur base du poids de la préparation totale ; et **en ce que** la pâte anthelminthique est dépourvue de tensioactif.

2. Pâte selon la revendication 1, **caractérisée en ce que** la lactone macrocyclique est de formule 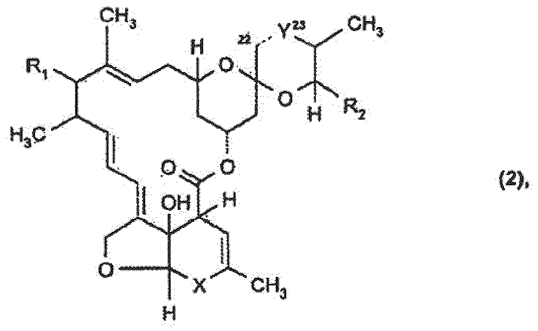 dans laquelle, X est -C(H)(OH)- ; -C(O)- ; ou -C(=N-OH)- ; Y est -C(H₂)- ; =C(H)- ; -C(H)(OH)-; ou -C(=N-OCH₃)- ; R₁ est hydrogène ou un des radicaux 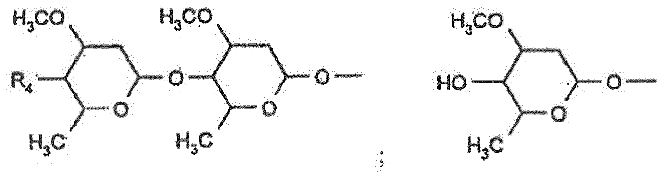 ou 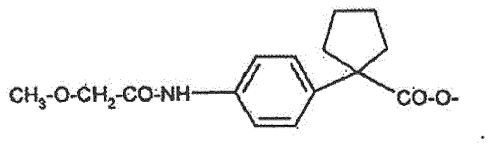 R₄ est hydroxyle, -NH-CH₃ ou -NH-OCH₃ et R₂ est hydrogène, -CH₃, -C₂H₅, -CH (CH₃)₂, -CH(CH₃)-C₂H₅, -C(CH₃)=CH-CH(CH₃)₂ ou cyclohexyle ; et, si la liaison entre les atomes 22 et 23 représente une double liaison, l'atome de carbone en position 23 est non substitué de sorte que Y est =C(H)-, ou si la liaison entre les atomes 22 et 23 représente une liaison unique, l'atome de carbone en position 23 est non substitué ou substitué par hydroxy ou par le groupe N-O-CH₃ de sorte que Y est -C(H₂)- ; -C(H)(OH)- ; ou -C(=N-OCH₃)- ; sous forme libre ou sous forme d'un sel physiologiquement acceptable.

3. Pâte selon la revendication 1 ou 2, **caractérisée en ce que** la lactone macrocyclique est l'ivermectine ou l'oxime de milbemycine, tout particulièrement l'oxime de milbemycine.

4. Pâte selon l'une quelconque des revendications 1 à 3 comprenant :
de 0,5 à 20% en poids de praziquantel ;
de 0,1 à 8% en poids d'une lactone macrocyclique ;
de 0,5 à 40% en poids d'une cyclodextrine ;
de 0,1 à 10,0% en poids d'un épaississant ; et
jusqu'à 100% en poids d'eau.

5. Pâte selon l'une quelconque des revendications 1 à 4 comprenant :
de 2,0 à 7,5% en poids de praziquantel ;
de 1,0 à 2,5% en poids d'une lactone macrocyclique ;
de 2,0 à 7,5% en poids d'une cyclodextrine ;
de 1,0 à 5,0% en poids d'un épaississant ; et
jusqu'à 100% en poids d'eau.

6. Pâte selon la revendication 4 ou 5, **caractérisée en ce que** la lactone macrocyclique est l'oxime de milbemycine.

7. Pâte selon l'une quelconque des revendications 1 à 6, comprenant un ou plusieurs composants supplémentaires choisis parmi le groupe composé d'humidifiants, de conservateurs, d'agents ajustant le pH, de colorants et d'aromatisants.

8. Pâte selon l'une quelconque des revendications 1 à 7, comprenant une teneur en eau de ≥60% en poids, sur base du poids de la préparation totale.

9. Pâte selon l'une quelconque des revendications 1 à 8, **caractérisée en ce que** l'épaississant est de la gomme xanthique ou est un mélange de gomme xanthique et de polyvinylpyrrolidone ou est un mélange de gomme xanthique, de polyvinyipyrrolidone et de cellulose microcristalline.

10. Pâte selon la revendication 2 composé
de 1,0 à 10% en poids de praziquantel ;
de 0,5 à 5% en poids d'oxime de milbemycine ;
de 1,0 à 20% en poids d'une cyctodextrine ;
de 0,5 à 7,5% en poids d'épaississants ;
de 1,0 à 10% en poids d'humidifiants ;
de 0,5 à 5% en poids de conservateurs ;
de 2,5 à 20% en poids d'aromatisants ;
de 0 à 5,0% en poids de colorants ; et
jusqu'à 100% en poids d'eau et d'acide.

11. Pâte selon l'une quelconque des revendications 1 à 11, **caractérisée en ce qu'**elle fait preuve d'une gravité spécifique comprise dans la gamme allant de 1,05 à 1,15.

12. Pâte selon l'une quelconque des revendications 1 à 11, **caractérisée en ce qu'**elle est thixotrope.

13. Procédé de fabrication d'une pâte anthelminthique selon l'une quelconque des revendications 1 à 12 consistant
(i) à préparer de l'eau et éventuellement d'autres ingrédients qui sont hydrosolubles ;
(ii) à ajouter de la cyclodextrine et à la disperser et/ou à la dissoudre partiellement dans l'eau ;
(iii) à ajouter le praziquantel et à le disperser dans le mélange eau/cyclodextrine;
(iv) à disperser la lactone macrocyclique, éventuellement d'autres ingrédients et un épaississant de façon consécutive ; et
(v) à maintenir la dispersion sous agitation sauf si une pâte homogène se forme.

14. Utilisation d'une pâte anthelminthique selon l'une quelconque des revendications 1 à 12 dans la fabrication d'un médicament destiné au traitement des endoparasites chez les animaux à sang chaud, tout particulièrement chez les chiens et les chats.

15. Utilisation d'une pâte anthelminthique selon l'une quelconque des revendications 1 à 12 dans la fabrication d'un médicament destiné au contrôle des endoparasites chez les animaux à sang chaud, tout particulièrement chez les chiens et les chats.
